# EUROPEAN PATENT SPECIFICATION

(11) **EP 2 743 258 B1**
(45) Date of publication and mention of the grant of the patent: **29.11.2017**
(21) Application number: 12822382.3
(22) Date of filing: 23.04.2012
(51) Int. Cl.: C07D 207/273

(54) **METHOD FOR PURIFYING LEVO-OXIRACETAM**
VERFAHREN ZUR AUFREINIGUNG VON LEVO-OXIRACETAM
PROCÉDÉ POUR PURIFIER LE LÉVO-OXIRACÉTAM

(30) Priority: 11.08.2011 CN 201110229931
(43) Date of publication of application: 18.06.2014
(73) Proprietor: CHONGQING RUZER PHARMACEUTICAL CO., LTD., Yubei District Chongqing 401120 (CN)
(72) Inventor: YE, Lei, Chongqing 401120 (CN); RONG, Zuyuan, Chongqing 401120 (CN)
(74) Representative: Patentgruppen A/S
(86) International application number: PCT/CN2012/074516
(87) International publication number: WO 2013/020389

(56) References cited:
- EP-A1- 0 223 328
- EP-A1- 2 573 071
- EP-A1- 2 743 259
- EP-A1- 2 743 260
- CN-A- 1 956 953
- CN-A- 101 367 757
- CN-A- 101 575 309
- CN-A- 102 050 774
- CN-A- 102 101 836
- CN-A- 102 249 974
- CN-A- 102 249 975

## Description

### Field of the Invention

This invention relates to a purification method by crystallization, in particular relates to a purification method of levo-4-hydroxy-2-oxo-1-pyrrolidine acetamide by crystallization.

### Description of the Prior art

Oxiracetam (Olaxiracetam) is a nootropic drug, which is firstly synthesized by Italy Smith Kline Beecham Ltd in 1974 and came into the market in 1987 in Italy. (S)-oxiracetam is a single enantiomer of oxiracetam, and the chemical name of which is (S)-4-hydroxy-2-oxo-1-pyrrolidine acetamide (hereinafter referred to as levo-oxiracetam for short).

Oxiracetam can promote the synthesis of phosphoryl choline and phosphoryl ethanolamine, and promote cerebral metabolism, shows stimulatory effects to specific central nervous pathway through blood-brain barrier, increases the ATP/ADP ratio in the brain and increases the synthesis of protein and nucleic acid in the brain, can improve learning and memory functions of patients with intelligence disorder, and the drug itself have no direct vascular activity, nor the central excitation, its influence on learning and memory abilities is a persistent promoting effect.

CN1513836, CN1948285 and CN101121688 respectively reports a process for synthesis of racemate of two isomers levo-oxiracetam and dextro-oxiracetam; CN101367757 and CN101575309 respectively reports a method of the preparation of levo-oxiracetam; CN1424034, CN1555794, CN1562000 and CN101152175 respectively reports a method of the preparation of a injection formulation of oxiracetam, a dispersible tablet of oxiracetam, a lyophilized formulation of oxiracetam and a novel formulation of oxiracetam; WO93/06826 discloses that levo-oxiracetam has good therapeutic effects on the improvement of intelligence. However, currently the process for the purification of levo-oxiracetam is complicated or the purity of the product is not higher enough, thereby it is difficult to prepare levo-oxiracetam with high purity under low cost and simple process condition.

### Summary of the Invention

One objective of the invention is to provide a purification method of levo-oxiracetam. The purification method is simple and has low cost, and the levo-oxiracetam prepared by which contains low impurity and is in high purity.
The objective of the present invention is achieved by the following technical solutions:

A purification method of levo-oxiracetam, characterized in that:
(1) dissolving crude levo-oxiracetam in water to form a solution, adding an organic solvent dropwise to the solution until the solution just turns turbid to obtain a mixture, wherein the organic solvent is ethanol, propanol or isopropanol, then the mixture is allowed to stand at a temperature of 0 to 18°C for 1 to 3 days, and colorless transparent crystals precipitate;
(2) filtering and washing with ice water of 0 to 5°C;
(3) vacuum drying to obtain levo-oxiracetam with high purity.
In order to allow the levo-oxiracetam dissolved in the water to precipitate sufficiently, the mass volume ratio (g/mL) of crude levo-oxiracetam to water in step (1) described above is preferably 1:0.5 to 1:2, and further preferably 1:0.75; the mixture obtained in step (1) described above stands preferably at a temperature of 5 to 15°C, and further preferably 9°C.

In order to further improve the purity of the levo-oxiracetam obtained ultimately, the ice water used in step (2) described above is 1 to 3°C, and further preferably 2°C; the mass volume ratio (g/mL) of crystals precipitating in step (1) described above to ice water is preferably 1:1 to 1:2. Further preferably, the vacuum drying in step (3) described above is carried out at 26 to 28°C for 4 to 5 hours.

In particular, a purification method of levo-oxiracetam is performed by following steps:
(1) dissolving crude levo-oxiracetam (purity≤89%) in water to form a solution, adding propanol dropwise to the solution until the solution just turns turbid to obtain a mixture, then the mixture is allowed to stand at a temperature of 9°C for 29 to 30 hours, and colorless transparent crystals precipitate; mass volume ratio (g/mL) of the crude levo-oxiracetam to water is 1:0.75;
(2) filtering the colorless transparent crystals precipitated in above steps and washing with ice water; mass volume ratio (g/mL) of the colorless transparent crystals precipitated in the step (1) to ice water is ranging from 1:1 to 1:2, and the temperature of the ice water is 2°C;
(3) Then vacuum drying at a temperature of 26 to 28°C for 4 to 5 hours to obtain levo-oxiracetam with high purity.
The present invention has the following advantages:
It is not difficult for a person skilled in the art to know that levo-oxiracetam has good water solubility and can dissolve into water rapidly to form a solution, therefore people always consider it is impracticable that purifying levo-oxiracetam by using water as solvent. With conventional ideas, the inventor has adopted organic solvents to purify levo-oxiracetam, and conducted numerous experimental studies to continually explore various steps and process conditions, however the purity of levo-oxiracetam obtained finally cannot meet the desirable requirement. The inventor discovered accidentally that in one of the long-term experiments, adopting a purification process using water and organic solvent can effectively improve the purity of crude levo-oxiracetam from 89% to 97.5-98.2% of HPLC purity, which greatly improves the purity of levo-oxiracetam; meanwhile the method of the invention is simple with mild controlling conditions, low cost, environmentally friendly without the pollution caused by organic solvent, and is very suitable for large-scale manufacture.

### Detailed Description of the Preferred Embodiments

### Example 1

A purification method of levo-oxiracetam was performed by the following steps:

1g of crude levo-oxiracetam (89% purity) was dissolved in 0.75 mL of water to form a solution. Add Organic solvent propanol dropwise to the solution until the solution just turned turbid to obtain a mixture. Then the mixture was allowed to stand at 9°C for 30 hours, and colorless transparent crystals precipitated. The colorless transparent crystals were filtered, washed with 2mL of ice water of 2°C from top and dried under vacuum at 28°C for 4.5 hours to obtain 0.5g of colorless crystalline product in HPLC purity of 98.2%.

The preparation of the crude levo-oxiracetam described above is performed by the following steps:
(a), 28.50g of glycinamide hydrochloride, 20.65g of sodium bicarbonate and 200mL of anhydrous ethanol were charged into a three-necked round-bottom flask to form a mixture, and the pH value was maintained at about 7.4. Then the mixture was heated to reflux with stirring;
(b) After refluxed for 2 hours, 39.08g of ethyl (S) 4-chloro-3-hydroxybutyrate was added into the mixture dropwise. During the process of addition, another 20.65g of sodium bicarbonate was added in five portions, and the amount of the base added in each portion was controlled by detecting the pH value to maintain it ≤ 8.5;
(c) The reaction mixture was further refluxed for 24 hours after the addition of ethyl (S) 4-chloro-3-hydroxybutyrate was completed. The reaction was terminated until the content of levo-4-hydroxy-2-oxo-1-pyrrolidine acetamide measured by HPLC was 74%. The solution obtained was thermal filtered and concentrated to obtain crude product of levo-4-hydroxy-2-oxo-1-pyrrolidine acetamide;
(d) The obtained crude product was dissolved in 50mL of water to obtain a solution, the solution was treated with 500mL of 001x7 strong acid styrene type cation exchange resin and the fractions containing products were collected. The collected aqueous solution was neutralized with 201x7 strong base styrene type anion exchange resin. The neutralization was determined as completed until the measured pH value of the solution reached 7.0±0.1.

### Example 2

A purification method of levo-oxiracetam was performed by the following steps:

6L of water was added to dissolve 5kg of crude levo-oxiracetam (89% purity) and formed a solution, then an organic solvent such as ethanol, propanol or isopropanol was added dropwise until the solution just turned turbid to form a mixture, then the mixture was allowed to stand at 12°C for 29 hours, and colorless transparent crystals precipitated. The colorless transparent crystals precipitated were filtered, washed with 4L of ice water of 5°C from top and dried under vacuum at room temperature for 5 hours to obtain 2.5kg of colorless crystalline product in HPLC purity of 98%.

### Example 3 (not part of the invention)

A purification method of levo-oxiracetam was performed by the following steps:

1.5L of water was added to dissolve 1 kg of crude levo-oxiracetam (89% purity) to obtain a solution, then an organic solvent THF was added dropwise until the solution just turned turbid to form a mixture, then the mixture was allowed to stand at 6°C for 30 hours, and colorless transparent crystals precipitated. The colorless transparent crystals precipitated were filtered, washed with 0.5 L of ice water of 1°C and dried under vacuum at 28°C for 4 hours to obtain 0.4 kg of colorless crystalline products in HPLC purity of 97.8%.

## Claims

1. A purification method of levo-oxiracetam, **characterized in that**, performing by following steps:
(1) dissolving crude levo-oxiracetam in water to form a solution; adding an organic solvent dropwise to the solution until the solution just turns turbid to obtain a mixture, wherein the organic solvent is ethanol, propanol or isopropanol; and wherein the mixture is allowed to stand at a temperature of 0 to 18°C for 1 to 3 days, and colorless transparent crystals precipitate;
(2) filtering and washing with ice water of 0 to 5°C;
(3) vacuum drying to obtain levo-oxiracetam with high purity.

2. The purification method of levo-oxiracetam according to Claim 1, **characterized in that**: mass volume ratio (g/mL) of the crude levo-oxiracetam to the water in the step
(1) is ranging from 1:0.5 to 1:2, the temperature for standing of the mixture is from 5 to 15°C.

3. The purification method of levo-oxiracetam according to Claim 2, **characterized in that**: the mass volume ratio (g/mL) of the crude levo-oxiracetam to the water in the step (1) is 1:0.75, the temperature for standing of the mixture is 9°C.

4. The purification method of levo-oxiracetam according to any one of Claims 1 to 3, **characterized in that**: the ice water used in the step (2) is 1 to 3°C; the mass volume ratio (g/mL) of the crystals precipitated in the step (1) to the ice water is ranging from 1:1 to 1:2.

5. The purification method of levo-oxiracetam according to Claim 4, **characterized in that**: the ice water used in the step (2) is 2°C.

6. The purification method of levo-oxiracetam according to Claim 5, **characterized in that**: the vacuum drying in the step (3) is carried out at a temperature from 26 to 28°C for 4 to 5 hours.

7. The purification method of levo-oxiracetam according to Claim 1 comprising the following steps:
(1) dissolving the crude levo-oxiracetam (≤ 89% purity) in water to form a solution; adding propanol dropwise to the solution until the solution just turns turbid to obtain a mixture, then the mixture is allowed to stand at a temperature of 9°C for 29 to 30 hours, and colorless transparent crystals precipitate; mass volume ratio (g/mL) of the crude levo-oxiracetam to the water is 1:0.75;
(2) filtering the colorless transparent crystals precipitated and washing with ice water, mass volume ratio (g/mL) of the colorless transparent crystals precipitated in the step (1) to the ice water is ranging from 1:1 to 1:2, the temperature of the ice water is 2°C;
(3) then drying under vacuum at a temperature of 26 to 28°C for 4 to 5 hours to obtain levo-oxiracetam with high purity.

## Patentansprüche

1. Reinigungsverfahren von Levo-Oxiracetam, **dadurch gekennzeichnet, dass** es durch die folgenden Schritte ausgeführt wird:
(1) Lösen von rohem Levo-Oxiracetam in Wasser, um eine Lösung zu bilden; tropfenweises Hinzugeben eines organischen Lösungsmittels, bis die Lösung gerade trüb wird, um eine Mischung zu erhalten, wobei das organische Lösungsmittel Ethanol, Propanol oder Isopropanol ist; und wobei man die Mischung bei einer Temperatur von 0 bis 18°C 1 bis 3 Tage stehen lässt und farblose transparente Kristalle ausfallen;
(2) Abfiltrieren und Waschen mit Eiswasser von 0 bis 5°C;
(3) Vakuumtrocknen, um Levo-Oxiracetam mit hoher Reinheit zu erhalten.

2. Reinigungsverfahren von Levo-Oxiracetam nach Anspruch 1, **dadurch gekennzeichnet, dass**: das Masse-Volumen-Verhältnis (g/ml) des rohen Levo-Oxiracetam zu dem Wasser in dem Schritt (1) im Bereich von 1:0,5 bis 1:2 liegt, die Temperatur für das Stehenlassen der Mischung 5 bis 15°C beträgt.

3. Reinigungsverfahren von Levo-Oxiracetam nach Anspruch 2, **dadurch gekennzeichnet, dass**: das Masse-Volumen-Verhältnis (g/ml) des rohen Levo-Oxiracetam zu dem Wasser in dem Schritt (1) 1:0,75 beträgt, die Temperatur für das Stehenlassen der Mischung 9°C beträgt.

4. Reinigungsverfahren von Levo-Oxiracetam nach einem der Ansprüche 1 bis 3, **dadurch gekennzeichnet, dass**: das in dem Schritt (2) verwendete Eiswasser 1 bis 3°C hat; das Masse-Volumen-Verhältnis (g/ml) der in dem Schritt (1) ausgefällten Kristalle zu dem Eiswasser im Bereich von 1:1 bis 1:2 liegt.

5. Reinigungsverfahren von Levo-Oxiracetam nach Anspruch 4, **dadurch gekennzeichnet, dass**: das in dem Schritt (2) verwendete Eiswasser 2°C hat.

6. Reinigungsverfahren von Levo-Oxiracetam nach Anspruch 5, **dadurch gekennzeichnet, dass**: das Vakuumtrocknen in dem Schritt (3) bei einer Temperatur von 26 bis 28°C für 4 bis 5 Stunden ausgeführt wird.

7. Reinigungsverfahren von Levo-Oxiracetam nach Anspruch 1, welches die folgenden Schritte umfasst:
(1) Lösen des rohen Levo-Oxiracetam (≤ 89% Reinheit) in Wasser, um eine Lösung zu bilden; tropfenweises Hinzugeben von Propanol zu der Lösung, bis die Lösung gerade trüb wird, um eine Mischung zu erhalten, dann lässt man die Mischung bei einer Temperatur von 9°C für 29 bis 30 Stunden stehen und farblose transparente Kristalle fallen aus; das Masse-Volumen-Verhältnis (g/ml) des rohen Levo-Oxiracetam zu dem Wasser beträgt 1:0,75;
(2) Abfiltrieren der ausgefällten farblosen transparenten Kristalle und Waschen mit Eiswasser, wobei das Masse-Volumen-Verhältnis (g/ml) der in dem Schritt (1) ausgefällten farblosen transparenten Kristalle zu dem Eiswasser im Bereich von 1:1 bis 1:2 liegt, die Temperatur des Eiswassers 2°C beträgt;
(3) dann Trocknen unter Vakuum bei einer Temperatur von 26 bis 28°C für 4 bis 5 Stunden, um Levo-Oxiracetam mit hoher Reinheit zu erhalten.

## Revendications

1. Procédé de purification de lévo-oxiracétam, **caractérisé en ce qu'**il est mené à bien par les étapes suivantes :
(1) dissolution de lévo-oxiracétam brut dans de l'eau pour former une solution ; ajout d'un solvant organique goutte à goutte à la solution jusqu'à ce que la solution devienne trouble pour obtenir un mélange, dans lequel le solvant organique est l'éthanol, le propanol ou l'isopropanol ; et dans lequel on laisse reposer le mélange à une température de 0 à 18°C pendant 1 à 3 jours, et des cristaux incolores transparents précipitent ;
(2) filtration et lavage avec de l'eau glacée à 0 à 5 °C ;
(3) séchage sous vide pour obtenir le lévo-oxiracétam de pureté élevée.

2. Procédé de purification de lévo-oxiracétam selon la revendication 1, **caractérisé en ce que** : le rapport masse/volume (g/ml) du lévo-oxiracétam brut sur l'eau dans l'étape (1) se trouve dans la plage de 1/0,5 à 1/2, la température de repos du mélange est de 5 à 15 °C.

3. Procédé de purification de lévo-oxiracétam selon la revendication 2, **caractérisé en ce que** : le rapport masse/volume (g/ml) du lévo-oxiracétam brut sur l'eau dans l'étape (1) est de 1/0,75, la température de repos du mélange est de 9°C.

4. Procédé de purification de lévo-oxiracétam selon l'une quelconque des revendications 1 à 3, **caractérisé en ce que** : l'eau glacée utilisée à l'étape (2) est à 1 à 3°C ; le rapport masse/volume (g/ml) des cristaux précipités à l'étape (1) sur l'eau glacée se trouve dans la plage de 1/1 à 1/2.

5. Procédé de purification de lévo-oxiracétam selon la revendication 4, **caractérisé en ce que** : l'eau glacée utilisée à l'étape (2) est à 2°C.

6. Procédé de purification de lévo-oxiracétam selon la revendication 5, **caractérisé en ce que** : le séchage sous vide à l'étape (3) est réalisé à une température de 26 à 28°C pendant 4 à 5 heures.

7. Procédé de purification de lévo-oxiracétam selon la revendication 1, comprenant les étapes suivantes :
(1) dissolution de lévo-oxiracétam brut (pureté ≤ 89 %) dans de l'eau pour former une solution ; ajout de propanol goutte à goutte à la solution jusqu'à ce que la solution devienne trouble pour obtenir un mélange, avant de laisser le mélange reposer à une température de 9 °C pendant 29 à 30 heures, et des cristaux incolores transparents précipitent ; le rapport masse/volume (g/ml) du lévo-oxiracétam brut sur l'eau est de 1/0,75 ;
(2) filtration des cristaux incolores transparents et lavage avec de l'eau glacée, le rapport masse/volume (g/ml) des cristaux incolores transparents précipités à l'étape (1) sur l'eau glacée se trouve dans la plage de 1/1 à 1/2, la température de l'eau glacée est de 2 °C ;
(3) puis le séchage sous vide à une température de 26 à 28 °C pendant 4 à 5 heures pour obtenir du lévo-oxiracétam de pureté élevée.
